# EUROPEAN PATENT APPLICATION

(11) **EP 3 954 768 A2**
(43) Date of publication of application: **16.02.2022**
(21) Application number: 21190849.6
(22) Date of filing: 11.08.2021
(51) Int. Cl.: C12N 9/02, C12N 9/10, C12N 9/88, C12N 15/70, C12P 7/22

(54) **ENGINEERING BACTERIA FOR FERULIC ACID PRODUCTION, PREPARATION METHOD AND USE THEREOF**

(30) Priority: 12.08.2020 CN 202010806606
(71) Applicant: CAS Center for Excellence in Molecular Plant Sciences, Shanghai 200032 (CN)
(72) Inventor: WANG, Yong, SHANGHAI (CN); LV, Huajun, SHANGHAI (CN); ZHANG, Ying, SHANGHAI (CN); SHAO, Jie, SHANGHAI (CN); LIU, Haili, SHANGHAI (CN)
(74) Representative: EP&C

(57) **Abstract**

The disclosure provides an engineering bacterium for ferulic acid production, a preparation method of the bacterium and use thereof. The invention provides an engineering bacterium that can efficiently produce ferulic compounds by expressing a series of heterologous enzymes in a host cell through gene recombination technology. The expression system constructed by the invention has low metabolic background, strong heterologous expression ability and low cost. The system can synthesize the end product through relatively simple steps, and provide a new way for the industrial production of ferulic acid, intermediates or derivatives thereof.

## Description

### FIELD OF DISCLOSURE

The disclosure belongs to the technical field of synthetic biology and industrial biology. More specifically, the disclosure relates to a recombinant cell for efficient synthesis of ferulic acid and a preparation method and use thereof.

### BACKGROUND OF DISCLOSURE

Plant secondary metabolites have attracted much attention for centuries because of their potential advantages. However, it faces the problems of difficult production and high cost.

Ferulic acid (FA) is an antioxidant naturally presents in plant cell walls, has functions like anti-inflammatory, and acts as a free radical scavenger. It is the main effective component of Angelica sinensis, Ligusticum chuanxiong Hort. FA is widely used in food, cosmetic and medicine areas. Extracting FA from plant materials rich in FA, such as Angelica sinensis, ferula, Ligusticum chuanxiong Hort., wheat bran and rice bran, is the main source of medicinal or commercial FA, but it costs high to treat the industrial waste. Chemical synthesis of FA from vanillin and malonic acid obtains the products of mixture of trans- and cis- ferulic acids, that makes it hard to carry out separation for cis-ferulic acids. In addition, based on biotechnology methods, FA can be transformed from ferulic acid precursors such as eugenol, lignin and phenols by some microorganisms or enzymes and renewable resources, which is efficient and clean. However, the process is not mature and needs further research.

Although the diversity of microorganism and microbial genome data provides some potentially useful gene resources for the development of efficient microbial artificial pathways, how to effectively select and utilize them is still an urgent research in this field.

Patent application CN201510234157.5 (disclosed on September 23, 2015) described a construction method and biotransformation method of an engineering bacterium for ferulic acid production. For the production, eugenol is used as precursor and a strong promoter is used to co-express the enzyme coupling system including vanillin oxidase, coniferol dehydrogenase and coniferyl aldehyde dehydrogenase genes in *E. coli.* At the same time, it is coupled with xylose reductase to reduce xylose and regenerate nicotinamide adenine dinucleotide and nicotinamide adenine dinucleotide phosphate, so as to maintain the coenzyme balance in ferulic acid biotransformation system, and generate ferulic acid by catalyzing eugenol in the whole cell.

The field still needs researches on the biosynthesis of FA and improves the biosynthesis process to solve the problems in FA production, improve the efficiency and reduce the cost of FA production.

### SUMMARY OF DISCLOSURE

The disclosure provides an engineering bacterium for ferulic acid production, a preparation method of the bacterium and use thereof.

The disclosure also aims to provide a fermentation method for high-yield ferulic acid.

The first aspect of the present disclosure provides a recombinant cell for ferulic acid production, wherein the cell expresses the following exogenous enzymes: Tyrosine ammonia-lyase (TAL), 4-coumarate-3-hydroxylase (SAM5) and caffeic acid O-methyltransferase (COMT).

In a preferred embodiment, the recombinant cell does not contain the following exogenous enzymes or proteins: PEPS, DAHPS, CM/PDH, ECH.

In another preferred embodiment, the recombinant cell also expresses a exogenous enzyme: pyridine nucleotide transhydrogenase (pntAB).

In another preferred embodiment, the tyrosine ammonia-lyase is from *Rhodobacter sphaeroides, Streptomyces albus, Rhodobacter capsulatus, Micromonospora echinofusca.*

In another preferred embodiment, the 4-coumarate-3-hydroxylase is from *Saccharothrix espanaensis, Streptomyces lunaelactis, Nocardia farcinica, Rhodococcus ruber.*

In another preferred embodiment, the caffeic acid O-methyltransferase is from *Triticum aestivum, Hordeum vulgare, Festuca arundinacea, Lolium perenne.*

In another preferred embodiment, the pyridine nucleotide transhydrogenase is from *Escherichia coli (Escherichia coli MG1655).*

In another preferred embodiment, the recombinant cell comprises a prokaryotic cell or a eukaryotic cell; preferably, the prokaryotic cell comprises *E. coli, Bacillus subtilis* or *Streptomyces,* or the eukaryotic cell comprises a fungal cell, a yeast cell, an insect cell or a mammalian cells; more preferably, the recombinant cell is *E. coli*; more preferably, the *E. coli* is JM109 (DE3).

In another preferred embodiment, in the expression cassette(s) of tyrosine ammonia-lyase, 4-coumarate-3-hydroxylase, caffeic acid O-methyltransferase, the promoter(s) comprises a promoter selected from the group consisting of: a T7 promoter, a T5 promoter, a trc promoter; preferably comprises a T7 promoter.

In another preferred embodiment, the replicon(s) comprises a replicon selected from the group consisting of: pBR322, p15a, pSC101; preferably, comprises pBR322.

In another preferred embodiment, the recombinant cell does not simultaneously comprise an enzyme expression cassette with p15a as a replicon and an enzyme expression cassette with T5 as a promoter.

In another preferred embodiment, tyrosine ammonia-lyase, 4-coumarate-3-hydroxylase, and caffeic acid O-methyltransferase are linked in tandem in the same expression cassette, or are placed in different expression cassettes, respectively; preferably, they are linked in tandem in the same expression cassette.

In another preferred embodiment, in the expression cassette of the pyridine nucleotide transhydrogenase, the promoter(s) comprises a promoter selected from the group consisting of: a T7 promoter, a T5 promoter; preferably comprises a T7 promoter.

In another preferred embodiment, in the expression cassette of the pyridine nucleotide transhydrogenase, the operon comprises an operon selected from T7 operon and T5 operon.

In another preferred embodiment, in the expression cassette of the pyridine nucleotide transhydrogenase, the replicon comprises SC101.

Another aspect of the disclosure provides a use of the recombinant cell for the production of ferulic acid; preferably, for the conversion of L-tyrosine to ferulic acid.

Another aspect of the disclosure provides a method for producing ferulic acid, wherein the method comprises: (1) providing the recombinant cell according to any one of the above embodiments; and (2) culturing the recombinant cell of (1) to produce ferulic acid.

In a preferred embodiment, the recombinant cell is a prokaryotic cell using glycerol as a carbon source to produce ferulic acid; preferably, the culture medium of the cell comprises glycerol of 0.5% to 8% by volume, preferably 1% to 6%, more preferably 1.5% to 4%, such as 1.8%, 2%, 2.5%, 3%, 3.5%, etc.

In another preferred embodiment, in step (2), said culturing the recombinant cell of (1) is in a culture system containing L-tyrosine.

In another preferred embodiment, the recombinant cell is a prokaryotic cell and the culturing is in a medium comprising a basic medium selected from (but not limited to) M9Y medium, TB medium, or LB medium.

In another preferred embodiment, the culture temperature of the recombinant cell is 25°C to 38°C, such as 26°C, 28°C, 30°C, 32°C, 34°C, 36°C, 37°C.

In another preferred embodiment, the recombinant cell is cultured at 100 rpm to 400 rpm, preferably 150 rpm to 350 rpm, more preferably 200 rpm to 300 rpm, such as 250 rpm.

In another preferred embodiment, the recombinant cell is a prokaryotic cell, preferably *E. coli.* Its expression is induced by IPTG.

In another preferred embodiment, the fermentation of the recombinant cell is carried out for 1 to 20 days, preferably 2 to 15 days, more preferably 2.5 to 10 days (such as 3, 4, 5, 6, 7, 8 and 9 days).

In another preferred embodiment, the concentration of L-tyrosine in the culture system is 0.1g/L to 50g/L, preferably 0.2g/L to 40g/L, more preferably 0.4g/L to 20g/L. The concentration in the culture system is, for example, 0.3 g/L, 0.5 g/L, 0.8 g/L, 1 g/L, 1.2 g/L, 1.5 g/L, 2 g/L, 3 g/L, 5 g/L, 10 g/L, 15 g/L, 30 g/L, etc.

In another preferred embodiment, the L-tyrosine is catalyzed by the tyrosine ammonia-lyase to form p-coumaric acid; the p-coumaric acid is catalyzed by the 4-coumarate-3-hydroxylase to form caffeic acid; the caffeic acid is catalyzed by caffeic acid O-methyltransferase to form ferulic acid.

Another aspect of the disclosure provides an expression cassette or recombinant construct (such as an expression vector) comprising nucleic acids encoding a group of enzymes comprising tyrosine ammonia-lyase, 4-coumarate-3-hydroxylase and caffeic acid O-methyltransferase; preferably, the group of enzymes also comprises pyridine nucleotide transhydrogenase.

In another preferred embodiment, in the expression cassette(s) of tyrosine ammonia-lyase, 4-coumarate-3-hydroxylase, caffeic acid O-methyltransferase, the promoter(s) comprises a promoter selected from the group consisting of: a T7 promoter, a T5 promoter, a trc promoter; preferably comprises a T7 promoter; or the replicon(s) comprises a replicon selected from the group consisting of: pBR322, p15A, pSC101; preferably comprises pBR322.

In another preferred embodiment, in the expression cassette of pyrimidine nucleotide hydrogenase, the promoter(s) comprises a promoter selected from the group consisting of: a T7 promoter, a T5 promoter; preferably comprises a T7 promoter; the replicon(s) comprises a replicon selected from the group consisting of: SC101, p15a, pBR322.

Another aspect of the disclosure provides a use of the expression cassette or recombinant construct in the manufacture of a recombinant cell for producing ferulic acid.

Another aspect of the disclosure provides a use of a group of enzymes or the coding nucleic acids or expression cassette of the group of enzymes, for expressing and producing ferulic acid in a recombinant cell; or in the manufacture of a recombinant cell for producing ferulic acid; the group of enzymes comprises tyrosine ammonia-lyase, 4-coumarate-3-hydroxylase and caffeic acid O-methyltransferase; preferably, the group of enzymes also comprises pyridine nucleotide transhydrogenase.

Another aspect of the disclosure provides a kit for the production of ferulic acid, which comprises the recombinant cell.

Another aspect of the disclosure provides a kit for the production of ferulic acid, which comprises the expression cassette or recombinant construct; preferably, the kit also comprises a host cell.

In a preferred embodiment, the host cell comprises a prokaryotic cell or a eukaryotic cell; preferably, the prokaryotic cell comprises *E. coli, Bacillus subtilis* or *Streptomyces,* or the eukaryotic cell comprises a fungal cell, a yeast cell, an insect cell or a mammalian cells; more preferably, the host cell is *E. coli*; more preferably, the *E. coli* is JM109 (DE3).

In another preferred embodiment, the kit also comprises L-tyrosine and/or basic culture medium.

In another preferred embodiment, the basic culture medium comprises L-tyrosine; preferably the concentration of L-tyrosine in the culture medium is 0.1g/L to 50g/L, preferably 0.2g/L to 40g/L, more preferably 0.4g/L to 20g/L. The concentration in the culture system is, for example, 0.3 g/L, 0.5 g/L, 0.8 g/L, 1 g/L, 1.2 g/L, 1.5 g/L, 2 g/L, 3 g/L, 5 g/L, 10 g/L, 15 g/L, 30 g/L, etc.

In another preferred embodiment, the kit also includes an expression inducer, such as IPTG. Other aspects of the disclosure will be apparent to those skilled in the art based on the disclosure herein.

### BRIEF DESCRIPTION OF DRAWINGS

Figure 1. Biosynthetic pathway of ferulic acid in the disclosure.
   TAL: tyrosine ammonia-lyase (Ammonia-lyase), from *Rhodobacter sphaeroides*;
   SAM5: 4-coumarate-3-hydroxylase (β-Coumaric acid 3-hydroxylase), from *Saccharothrix espanaensis*;
   COMT: caffeic acid O-methyltransferase (Caffeic acid O-methyltransferase) from *Triticum aestivum.*
Figure 2. Schematic diagram of ferulic acid synthesis pathway plasmids with different combinations of replicons and promoters. T7/T5 represents T7 promoter or T5 promoter, and pBR322/p15Aori represents pBR322 or p15A replicon.
Figure 3. The yield of ferulic acid (FA) with different amount of carbon source was detected. The experimental strain used T7 promoter and TB medium (containing 1g/L L-tyrosine). 3D represents day 3 and 5D represents day 5.
Figure 4. *E. coli* JM109 (DE3) or BL21 (DE3) was used as the host; plasmid pHJ697 (pBR322-T7-tal-sam5-comt); TB-2% glycerol-lg/L L-tyrosine medium.
Figure 5. The yield of ferulic acid using *E. coli* JM109 (DE3) as the host cell and using different replicons or promoters for expression respectively. TB medium (containing 2% glycerol and 1g/L L-tyrosine) was used.
Figure 6. The yield of ferulic acid after further expressing other enzymes (zwf, icd, gnd, pntAB or gapN) in *E. coli* JM109 (DE3) was compared with the control harboring an empty vector expression system without expressing said other enzymes.

### DETAILED DESCRIPTION

After in-depth research, a series of exogenous enzymes have been recombined and expressed in a host cell (preferably a prokaryotic host cell) through gene recombination, and an engineering bacterium that can efficiently produce ferulic acid is obtained. The disclosure also optimized the expression process of the engineering bacterium. The expression system constructed by the invention has low metabolic background, strong heterologous expression ability and low cost. The system can synthesize the end product, which is easy to separate, through relatively simple steps, and provide a new way for the industrial production of ferulic acid, intermediates or derivatives thereof.

### Term

As used herein, the "expression cassette" or "gene expression cassette" refers to a gene expression system containing all necessary elements required to express the target polypeptide, which usually includes the following elements: promoter, gene sequence encoding polypeptide, and terminator. In addition, it can optionally include signal peptide coding sequence, etc. These elements are operably linked.

As used herein, "operably linked (to)" or "operably connected (to)" is intended to mean a functional spatial arrangement between two or more nucleic acid regions or nucleic acid sequences. For example, a promoter region is "operatively linked" to the nucleic acid sequence of a target gene when the promoter region is placed at a specific position relative to the nucleic acid sequence so that the transcription of the nucleic acid sequence is guided by the promoter region.

As used herein, the "expression cassette" refers to a gene expression system containing all necessary elements required to express the target polypeptide (such as the enzymes of interest in the disclosure), which usually includes the following elements: promoter, gene sequence encoding polypeptide and terminator. In addition, it can optionally include signal peptide coding sequence, etc. These elements are operably linked.

As used herein, the "expression construct" refers to a recombinant DNA molecule that contains the desired nucleic acid coding sequence. An expression construct may contain one or more gene expression cassettes. The "construct" is usually contained in an expression vector.

As used herein, "exogenous" or "heterologous" refers to the relationship between two or more nucleic acid sequences or protein (polypeptide) sequences from different sources, or the relationship between nucleic acid sequences or protein sequences from different sources and host cells. For example, if the combination of a nucleic acid/protein and a host cell is usually not natural, the nucleic acid is exogenous to the host cell. A specific sequence is "exogenous" to the cell or organism into which it is inserted.

As used herein, the ferulic acid also includes ferulic compounds. The "ferulic acid compound" can also be a variant based on the compound ferulic acid disclosed in the disclosure. For example, the core structure of the compound remains unchanged, but the substitution of groups (such as aliphatic hydrocarbon groups containing 1-4 carbon atoms (preferably 1-2 carbon atoms) occurs at certain (such as 1-3, 1-2) positions.

### Enzyme combination and its expression system

In the disclosure, the efficient production of ferulic compounds in engineering bacteria is realized by transforming a group of enzymes into engineering cells (engineering bacteria). This group of enzymes includes tyrosine ammonia-lyase (TAL), 4-coumarate-3-hydroxylase (SAM5) and caffeic acid O-methyltransferase (COMT). In a preferred embodiment, the group of enzymes also includes pyridine nucleotide transhydrogenase (pntAB). The inventor unexpectedly found that the use of pntAB can greatly promote the production efficiency of ferulic acid and greatly improve its yield.

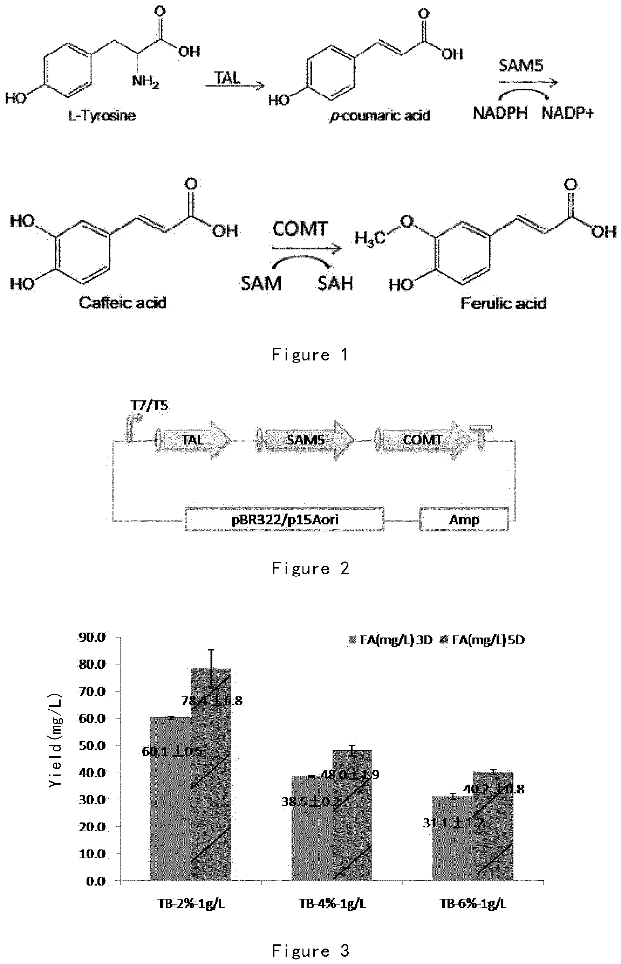

Based on the new discovery of the inventor, the disclosure provides a genetic engineering bacterium for producing ferulic compounds, in which the following exogenous enzymes: tyrosine ammonia-lyase (TAL), 4-coumarate-3-hydroxylase (SAM5) and caffeic acid O-methyltransferase (COMT) are expressed to realize the synthesis of ferulic acid in the engineering bacterium . Further, an exogenous pyridine nucleotide transhydrogenase (pntAB) was further expressed in the bacterium. In the engineering bacterium of the disclosure, the synthesis pathway of ferulic acid is as shown in Figure 1.

Genes encoding the enzymes herein may exist naturally. For example, it may be isolated or purified from animals, plants or microorganisms. In addition, the gene can also be artificially prepared, for example, the gene can be obtained by conventional genetic engineering recombination technology, or by synthetic method.

The sequences of the enzyme or its coding nucleic acid described in the disclosure can be those provided in Table 2 in Examples, or can be their variants or degenerate sequences. The "degenerate sequence" in the present disclosure refers to a nucleic acid sequence which encodes a protein with the same function but is different from the sequences provided in Table 2 in Examples. In the disclosure, the natural nucleic acid sequence encoding the enzyme or its codon-optimized variant can be used. The coding nucleic acid of the enzyme may comprise: the coding sequence encoding only the mature polypeptide; the coding sequence of the mature polypeptide and various additional coding sequence; the coding sequence of the mature polypeptide (and optionally additional coding sequence) and a non-coding sequence.

The disclosure also relates to a variant of the enzyme, which is different from its corresponding wild-type polypeptide in amino acid sequence, and is a single-position or multi-position variant, fragment, analog or derivative of the wild-type polypeptide. Such polynucleotide variant can be a naturally occurring allelic variant or an unnatural variant. These nucleotide variants include substitution variants, deletion variants and insertion variants. As known in the art, an allelic variant is an alternate form of a polynucleotide, which may be caused by one or more nucleotide substitutions, deletions or insertions, but does not substantially alter the function of the encoded polypeptide.

It should be understood that the yield of ferulic compounds can be further improved by well-known codon optimization methods, protein variant screening methods, or enzyme activity promotion methods in the art to optimize and construct an optimized engineering bacterium. These further optimization based on the embodiments of the disclosure shall also be covered by the technical solutions of the disclosure.

In a preferred embodiment, TAL is from *Rhodobacter sphaeroides,* SAM5 is from *Saccharothrix espanaensis* and COMT is from *Triticum aestivum.* The disclosure may also include the use of these enzymes from other microorganisms or plants, as long as they are highly homologous (e.g. having more than 80%, such as 85%, 90%, 95%, or even 98% sequence identity) with the enzymes described in Examples of the disclosure. Methods and tools for aligning sequence identity are also well known in the art, such as BLAST. The term "identity" refers to the level of similarity (i.e. sequence homology, similarity or identity) between two or more nucleic acids according to the percentage of identical positions.

Generally, the full-length coding sequence of each enzyme of the disclosure or fragments thereof can be obtained by PCR amplification, recombination or artificially synthetic methods. For the PCR amplification method, primers can be designed according to the relevant nucleotide sequences (especially the open reading frame sequences) described in the disclosure and the relevant sequences can be amplified. For longer sequences, two or more individual PCR amplifications are desired, which are followed by ligating the separately amplified fragments together in a proper order.

The disclosure also relates to a vector containing the coding nucleic acid and a host cell generated by genetic engineering with the vector.

In the disclosure, the sequence of the coding nucleic acid of each enzyme can be inserted into the recombinant expression vector. The term "recombinant expression vector" refers to bacterial plasmid, phage, yeast plasmid, plant cell virus, mammalian cell virus or other vectors well known in the art. In short, any plasmid or vector can be used, provided that it can replicate and be stable in the host. An important characteristic of an expression vector is that it usually contains an origin of replication, a promoter, a marker gene and a translation control element.

The nucleic acid sequence encoding each enzyme can be inserted into the recombinant expression vector respectively, and multiple recombinant expression vectors can be co-transformed into host cells. Expression cassettes of multiple genes can also be inserted into one recombinant expression vector in series and transformed into host cells. The recombinant expression vector can also include an expression regulatory sequence operably connected with the gene sequence to facilitate protein expression. It should be understood that those in the art can readily construct a recombinant expression vector based on the content of the disclosure. The obtained recombinant expression vector is also included in the scope of the disclosure.

For expression regulatory sequences or expression cassettes, inducible or constitutive promoters can be used according to different desire. Inducible promoters can realize more controllable protein expression and compound production, which is conducive to industrial application.

As a preferable embodiment of the disclosure, an expression cassette or recombinant construct (such as an expression vector) is provided, which comprises nucleic acids encoding a group of enzymes comprising tyrosine ammonia-lyase, 4-coumarate-3-hydroxylase and caffeic acid O-methyltransferase. In a more preferred embodiment, the group of enzymes also includes pyridine nucleotide transhydrogenase.

The expression vector (expression construct) can be constructed by the technology familiar to those skilled in the art. Based on the selected enzyme(s) and the cell system to be used for expression, those skilled in the art can prepare the expression construct. Gene sequences can be inserted into different expression constructs (such as expression vectors) or into the same expression construct, as long as the encoded polypeptides can be effectively expressed and activated after being transformed into cells.

Vectors containing the above appropriate gene sequences and appropriate promoters or regulatory sequences can be used to transform appropriate host cells so that they can express proteins. In the disclosure, the host cell may include a prokaryotic cell or a eukaryotic cell; preferably, the prokaryotic cell include *Escherichia coli, Bacillus subtilis* or *Streptomyces,* or the eukaryotic cell include a fungal cell, a yeast cell, an insect cell or a mammalian cell. In a more preferred embodiment, the host cell is *Escherichia coli,* most preferably *Escherichia coli* JM109 (DE3).

In a preferred embodiment, in the expression cassette(s) of tyrosine ammonia-lyase, 4-coumarate-3-hydroxylase, caffeic acid O-methyltransferase, the promoter(s) comprises a promoter selected from the group consisting of: a T7 promoter, a T5 promoter; preferably comprises a T7 promoter; or the replicon(s) comprises a replicon selected from the group consisting of: pBR322, p15a; preferably comprises pBR322. The inventor unexpectedly found that the engineering bacteria with the construct containing pBR322 combined with T7 promoter have the highest expression efficiency and ferulic acid yield.

In a preferred embodiment, in the expression cassette of pyrimidine nucleotide hydrogenase, the promoter(s) comprises a promoter selected from the group consisting of: a T7 promoter, a T5 promoter; preferably comprises a T7 promoter; or the replicon(s) comprises SC101, etc. The inventor unexpectedly found that the engineering bacteria with the construct containing SC101 replicon combined with T7 promoter have the highest expression efficiency and ferulic acid yield. It should be understood that in the present disclosure, the promoter or replicon also includes their functional variants, active fragments, etc. The disclosure also includes a nucleic acid with 80% or more (preferably more than 85%, more preferably more than 90%, most preferably more than 95%, such as 98%, 99%) identity with a sequence of any promoter or replicon of the disclosure, and the nucleic acid also has the synergistic function of the corresponding promoter or replicator.

The transformation of host cells with recombinant DNA can be carried out by conventional techniques well known to those skilled in the art. The obtained transformants can be cultured by conventional methods under conditions suitable for cell growth, and the used culture medium can be a culture medium well known in the art. In a preferred embodiment, the culture medium of the cells contains the precursor L-tyrosine.

The disclosure also provides a kit for biosynthesis of ferulic compounds, including: the recombinant cell (engineering bacterium) constructed according to the disclosure. Alternatively, the kit includes: the expression cassette or recombinant construct constructed by the disclosure; preferably, the kit also includes a host cell, so as to facilitate the operation by those skilled in the art.

In a more preferred embodiment, the kit also includes an instruction describing the method of the biosynthesis.

The recombinant cell obtained in the disclosure has low metabolic background, strong heterologous expression ability and low cost. The cell can synthesize the end product, which is easy to separate, through relatively simple steps. The disclosure solves the problems in the ferulic compound synthesis by traditional biological and chemical methods, provides a new way for the industrial production of ferulic compounds.

The recombinant engineering bacterium constructed in the disclosure has good adaptability with each externally introduced enzyme, presents good expression and catalytic activity without modification of the enzyme structure, and has development and application potential. It should be understood that the technical solution obtained by further improving the structure or function of the enzyme on the basis of the disclosure should also be included in the disclosure.

### Method for synthesizing ferulic acid

Based on the new discovery of the inventor, the disclosure provides a method for heterologous synthesis of ferulic acid by microorganisms. The method comprises: culturing the recombinant cells constructed according to the disclosure to produce ferulic acid. According to the technical solution of the disclosure, the synthetic pathway of ferulic compounds is shown in Figure 1.

In the synthesis pathway of the disclosure, L-tyrosine is used as the precursor, and the concentration of L-tyrosine in the culture system is 0.1g/L to 50g/L, preferably 0.2g/L to 40g/L, more preferably 0.4g/L to 20g/L. According to the different fermentation scales and fermentation conditions, those skilled in the art can also appropriately adjust the amount of the precursor, which is also included in the disclosure.

Based on the constructed strain and its expression, the inventor also systematically studied a series of factors to improve yield, including gene efficiency and suitability, gene dose and culture medium. On this basis, the inventor further optimized the process of ferulic compounds production.

In a preferred embodiment of the disclosure, *E. coli* is used as the engineering bacterium for recombinant expression, more preferably *E. coli* JM109 (DE3).

In another preferred embodiment of the disclosure, glycerol is used as a carbon source; more preferably, the engineering bacteria are cultured at a glycerol concentration of 1.5% to 4% by volume, more particularly about 2%. The basic medium used for fermentation can be a commercial medium, such as but not limited to M9Y medium, M9 medium, TB medium, LB medium, etc.

After obtaining the fermentation product, ferulic acid can be extracted from it by any technique known in the art. Well-known techniques such as high performance liquid chromatography can be used to analyze and identify the products to determine that the required compounds are obtained.

### The main advantages of the disclosure are:

The disclosure uses prokaryotic cells, especially *Escherichia coli,* to produce ferulic compounds, which not only solves the problems of consumption of a large amount of plant raw materials, limitation by seasonal and regional factors and low extraction efficiency in extraction of ferulic compounds from plants, but also avoids adverse factors in chemical synthesis, such as too many by-products, low activity of target products, serious environmental pollution and so on. Although prokaryotic cells are used in the disclosure, the problem of low activity of an enzyme when being heterologously expressed in prokaryotic cells is avoided. The disclosure is especially suitable for the efficient synthesis of ferulic compounds, and provides a new way for the industrial production of such compounds.

The results of the disclosure show that the metabolic pathway can produce ferulic compounds from low-cost substrates, which indicates that imitating the natural pathway is a good strategy for designing an artificial pathway.

The disclosure overcomes the disadvantages in the prior art such as high cost, environmental pollution and complex products, and provides a production method of ferulic acid with low cost, less pollution and single product.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Materials and methods

PCR product recovery and purification kit, Plasmid Extraction Kit, restriction endonuclease, nucleic acid and protein molecular weight standard, T4 ligase and related enzyme reaction buffer were purchased from New England biolab (NEB).

High fidelity DNA polymerase PrimeSTAR^{®} HS DNA Polymerase and PrimeSTAR^{®} Max DNA Polymerase kit were purchased from Takara.

The components of LB medium used for bacterial culture were purchased from Sigma.

Primer synthesis, TB medium, antibiotic and inducer isopropyl-β-D isothiogalactoside (IPTG) were purchased from Sangon Biotech (Shanghai) Co., Ltd.

The seamless cloning kit ClonExpress II One Step Cloning Kit was purchased from Vazyme Biotech (Nanjing) Co. Ltd.

The three genes *tal sam5* and *comt* of ferulic acid synthesis pathway were synthesized by Generay Biotech (Shanghai) Co., Ltd.

The primers used in Examples are shown in Table 1.

**Table 1**

| Primer | Sequence | SEQ ID NO: |
|---|---|---|
| T7 operatorF | cgggaattcgcgcaaaaaacccctcaag | 1 |
| T7 operatorR | ctttactaagctgacgatagtcatgccccg | 2 |
| pCL1920VF | gactatcgtcagcttagtaaagccctcgctag | 3 |
| pCL1920VR | gttttttgcgcgaattcccg | 4 |
| pro-VF | agatctcgatcctctacgc | 5 |
| pro-VR | tctagaaataattttgtttaactttaag | 6 |
| pro-T5-in-F | caaaattatttctagaatagttaatttctcctc | 7 |
| pro-T5-in-R | gtagaggatcgagatctaaatcataaaaaatttatttg | 8 |
| ori-p15a-inF | caaaagcaccgccggacatcagcgctagcggagtgtatactg | 9 |
| ori-p15a-inR | catgactaacatgagaattacaacttatatcgtatg | 10 |
| ori-VF | catacgatataagttgtaattctcatgttagtcatg | 11 |
| ori-VR | cagtatacactccgctagcgctgatgtccggcggtgcttttg | 12 |
| ECicd F | ccaagcttactagtttacatgttttcgatg | 13 |
| ECicd R | catgccatggaaagtaaagtagttg | 14 |
| ECgnd F | ccaagcttactagtttaatccagccattcg | 15 |
| ECgnd R | ggaattccatatgtccaagcaacagatc | 16 |
| ECzwfF | cccaagcttactagtttactcaaactcattccag | 17 |
| ECzwfR | catgccatggcggtaacgcaaacag | 18 |
| pntABHindF | cccaagcttactagtttacagagctttcaggattg | 19 |
| pntABNcoR | catgccatggaagggaatatcatg | 20 |
| gapNF | ctttaagaaggagatatacatgtttgaaaatatatcatcaaatggag | 21 |
| gapNR | gtgcggccgcaagcttgtcgattataggtttaaaactattgatttatg | 22 |
| GapN-V F | cataaatcaatagttttaaacctataatcgacaagcttgcggccgcac | 23 |
| GapN-VR | ctccatttgatgatatattttcaaacatgtatatctccttcttaaag | 24 |

### Example 1. Construction and optimization of engineering bacteria

In this Example, an engineering strain for ferulic acid production is constructed. The host strain is *E. coli* JM109 (DE3) or BL21 (DE3), which is transformed by plasmid(s) harboring the gene(s) of the ferulic acid biosynthetic pathway with tyrosine as a substrate.

The enzymes/genes used to construct the ferulic acid biosynthetic pathway gene expression cassette are shown in Table 2.

**Table 2**

| **Enzyme/Gene** | **Source** | GenBank Access No. |
|---|---|---|
| Tyrosine ammonia-lyase, TAL; EC 4.3.1.23 | *Rhodobacter sphaeroides* | GenBank_CP033447.1 |
| 4-coumarate-3-hydroxylase (β-Coumaric acid 3-hydroxylase, SAM5; EC 1.14.14.9) | *Saccharothrix espanaensis* | GenBank_HE804045.1 |
| Caffeic acid O-methytransferase, COMT; EC 2.1.1.68 | *Triticum aestivum* | GenBank_EF413031.1 |

The plasmid construction method includes the following steps:
1.1 Ferulic acid synthesis pathway genes linked into one operon
(1) Tyrosine ammonia-lyase gene (TAL, 1575 bp) synthesized by double digestion of *Nco* I and *Hind* III was cloned into pET21d vectors digested by *Nco* I and *Hind* III, and recombinant vectors pET21d-TAL were obtained. Similarly, 4-coumarate-3-hydroxylase gene (SAM5, 1542 bp) C3H enzyme gene (SAM5, 768 bp) and caffeic acid O-methyltransferase gene (COMT, 1074 bp) synthesized by double digestion with *Nde* I and *Hind* III were cloned into pET21a vectors digested by *Nde* I and *Hind* III, and recombinant vectors pET21a-SAM5 and pET21a-COMT were obtained.
(2) The recombinant vectors pET21d-TAL were digested with *Spe* I and *Hind* III and recovered as vector fragments. pET21a-SAM5 were digested with *Xba* I and *Hind* III, and recovered as 1587 bp fragments, which were linked with the vector fragments and transformed into cells. Therefore the recombinant vectors pET21d-TAL-SAM5 were obtained.
(3) The recombinant vectors pET21d-TAL-SAM5 were digested with *Spe* I and *Hind* III and recovered as vector fragments. pET21a-COMT were digested with *Xba* I and *Hind* III, and recovered as 1119 bp fragments, which were linked with the vector fragments and transformed into cells. Therefore the recombinant vectors pET21d-TAL-SAM5-COMT were obtained.
(4) By seamless splicing, the replicon pBR322 of vectors pET21d was replaced with p15a, and T7 promoter was replaced with T5 promoter.

Using plasmids pBR322-T7-tal-sam5-comt as templates, vector fragments were amplified with primer pro-VF/pro-VR. Using plasmid pQE30 as templates, the inserted fragments were amplified with primer pro-T5-in-F/pro-T5-in-R. T7 promoter was replaced with T5 promoter to construct pBR322-T5-tal-sam5-comt.

Similarly, the replicon was replaced with p15a, and p15a-T7-tal-sam5-comt and p15a-T5-tal-sam5-comt were constructed. Using plasmids pBR322-T7-tal-sam5-comt as templates, vector fragments were amplified with primer ori-VF/ori-VR. Using plasmid Pacyc184 as templates, the inserted fragments were amplified with primer ori-p15a-in-F/ori-p15a-in-R. pBR322 replicon was replaced with p15a replicon to construct p15a-T7-tal-sam5-comt. Using plasmids pBR322-T5-tal-sam5-comt as templates, vector fragments were amplified with primer ori-VF/ori-VR. Using plasmid Pacyc184 as templates, the inserted fragments were amplified with primer ori-p15a-in-F/ori-p15a-in-R. pBR322 replicon was replaced with p15a replicon to construct p15a-T5-tal-sam5-comt.

Recombinant vectors pBR322-T7-tal-sam5-comt, pBR322-T5-tal-sam5-comt, p15a-T7-tal-sam5-comt, p15a-T5-tal-sam5-comt were obtained. Figure 2 shows the schematic diagram of plasmids with different combinations of replicons and promoters.

The recombinant vectors were transformed into JM109 (DE3) to obtain engineering bacteria for fermentation. Fermentation conditions and methods: the culture medium was TB medium supplied with 2% glycerol and 1 g/L L-tyrosine; the primary bacteria were cultured overnight for 12 hours; then the cultured bacteria were trans-inoculated into new medium by 5%, initially induced by 0.1 mM IPTG and fermented at 28°C and 250 rpm, and sampled for determination at day 5.

After fermentation, the products were analyzed. It was preliminarily determined that the transformant with plasmid pBR322-T5-tal-sam5-comt was a high-yield strain.

### Example 2. Plasmid Optimization

### (1) Construction of plasmid pCL1920-T7 operator (T7 operon)

Using plasmids pCL1920 (Biovector, spectinomycin resistance, SC101 replicon) as templates, vector fragments were amplified with primer pCL1920VF/pCL1920VR. Using plasmid pET28a (kanamycin resistance, pBR322 replicon) as templates, the inserted fragments were amplified with primer T7 operatorF/T7 operatorR. The plasmid pCL1920-T7 operator was constructed by infusion method.

### (2) Construction of optimized plasmid

*Zwf* (*Nco*I*-Hind*III)*, gnd* (*Nde*I*-Hind*III)*,* and *icd* (*Nco*I*-Hind*III) were amplified using *E.coli* MG1655 (*Ec*) genome as template, digested with corresponding restriction endonucleases and linked into pET28a vectors by T4 ligase. Then, the inserted fragments were obtained by double digestion of the above vectors with *Xba*I*-Hind*III. The vector fragments were obtained by double digestion of pCL1920-T7 operator with *Xba*I*-Hind*III. Plasmids pCL1920-T7-*Ecicd*, pCL1920-T7-*Ecgnd*, and pCL1920-T7-*Eczwf* were constructed by linking the inserted fragments and the vector fragments using T4 ligase.

*pntAB* (*Nco*I*-Hind*III) were amplified using *E.coli* MG1655 (*Ec*) genome as template, digested with corresponding restriction endonucleases. Plasmids pCL1920-T7*-EcpntAB* were constructed by linking *pntAB* (*Nco*I*-Hind*III) into pCL1920-T7 operator vectors using T4 ligase.

*gap*N (GenBank access No. AE001437.1) from *Clostridium acetobutylicum* ATCC 824 was constructed into pCL1920-T7 operator by seamless splicing, to obtain the plasmid pCL1920-T7 operator-*CagapN*.

### Example 3: Determination of Carbon Source Amount

This Example used glycerol as the carbon source to study the amount of ferulic acid produced by different amounts of carbon source.

JM109 (DE3) strain transformed with pBR322-T7-tal-sam5-comt was tested for three treatment groups of 2% (V/V), 4% (V/V) and 6% (V/V) glycerol.

Monoclonal strain was selected and inoculated into 2 mL LB (10 ml small test tube) containing antibiotics, cultured overnight (12 h). The cultured bacteria were trans-inoculated by 5% into TB medium added with antibiotics, 0.1 mm IPTG and 1 g/L L-tyrosine, and fermented at 28 °C and 250 rpm, then sampled (1 ml) at day 3 and day 5. Samples were extracted twice with 1V ethyl acetate, re-suspended in methanol, and FA was detected by HPLC at 310 nm.

The results are shown in Figure 3. Unexpectedly, it is not that the higher the amount of carbon source, the better. The yield is significantly the highest with 2% glycerol.

### Example 4: Determination of Host

In this embodiment, the effects of different hosts on yield were compared between *E*. *coli* JM109 (DE3) and BL21 (DE3).

The host bacteria JM109 (DE3) and BL21 (DE3) were transformed with pBR322-T7-tal-sam5-comt respectively. The medium was TB medium supplied with 2% glycerol, 1 g/L L-tyrosine. The fermentation conditions were the same as previous Examples. Samples were taken for determination after 5 days of fermentation.

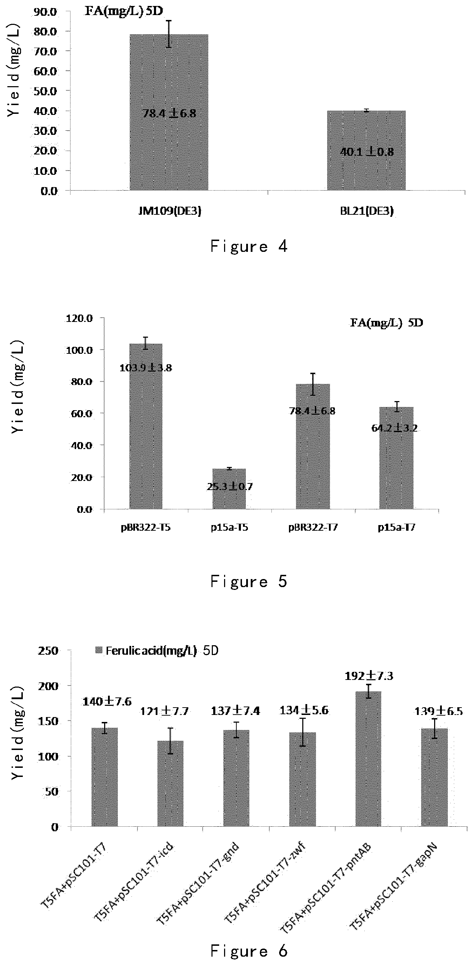

The results are shown in Figure 4. The yield of ferulic acid fermented by JM109 (DE3) is 78 mg/L, which is higher than that of 40 mg/L fermented by BL21 (DE3).

### Example 5: Determination of Replicon and Promoter

To optimize the product synthesis ability of the strains, pairwise combination of replicons pBR322 and p15A with promoters T7 and T5 was tested in host cells *E. coli* JM109 (DE3) for the effect of each element when applied to the expression system of the disclosure.

For this Example, hosts are JM109(DE3), and plasmids are pBR322-T7-tal-sam5-comt, pBR322-T5-tal-sam5-comt, p15a-T7-tal-sam5-comt, p15a-T5-tal-sam5-comt.

The medium was TB medium supplied with 2% glycerol, 1 g/L L-tyrosine. The fermentation conditions were the same as previous Examples. Samples were taken for determination after 5 days of fermentation.

Results are shown in Figure 5. The yield of strain JM109 (DE3)/pBR322-T5-tal-sam5-comt containing the combination of replicon and promoter pBR322-T5 was 104 mg/L, which is significantly higher than that of other groups.

### Example 6. Determination of the Effect of the Optimized Strains

In this Example, a series of genes are co-expressed by low copy vector to obtain the following strains:
T5FA+pSC101-T7:
   JM109(DE3)/pBR322-T5-tal-sam5-comt + pCL1920-T7 operator;
T5FA+pSC101-T7-icd:
   JM109(DE3)/pBR322-T5-tal-sam5-comt+ pCL1920-T7-*Ecicd*
T5FA+pSC101-T7-gnd:
   JM109(DE3)/pBR322-T5-tal-sam5-comt+ pCL1920-T7-*Ecgnd*
T5FA+pSC101-T7-zwf:
   JM109(DE3)/pBR322-T5-tal-sam5-comt+ pCL1920-T7-*Eczwf*
T5FA+pSC101-T7-pntAB:
   JM109(DE3)/pBR322-T5-tal-sam5-comt+pCL1920-T7-*EcpntAB*
T5FA+pSC101-T7-gapN:
   JM109(DE3)/pBR322-T5-tal-sam5-comt+ pCL1920-T7*-CagapN*

A relatively simple medium M9Y was used: containing NH₄Cl 4g/L, Na₂HPO₄ 6g/L, KH₂PO₄ 3g/L, NaCl 0.5g/L, MgSO₄·7H₂O 2mM, CaCl₂ 0.1mM, trace element solution 10 mL, yeast extract 0.5 g/L, glycerol 20 g/L, L-tyrosine 1 g/L. Trace element solution containing the following in g/L: H₃BO₃ 0.03, Thiamine 1, ZnCl₂ 0.94, CoCl₂ 0.5, CuCl₂ 0.38, MnCl₂ 1.6, FeCl₂ 3.6.

Results are shown in Figure 6. It can be seen that co-expression of zwf, icd, gnd or gapN did not significantly improve the ferulic acid yield of the engineering strain. However, co-expression of pntAB showed a significantly increased yield of 192 mg/L, which is unexpected.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A recombinant cell for ferulic acid production, wherein the cell expresses the following exogenous enzymes: tyrosine ammonia-lyase, 4-coumarate-3-hydroxylase and caffeic acid O-methyltransferase.

2. The recombinant cell according to claim 1, wherein the cell also expresses the following exogenous enzyme: pyridine nucleotide transhydrogenase.

3. The recombinant cell according to claim 1 or 2, wherein the tyrosine ammonia-lyase is from *Rhodobacter sphaeroides, Streptomyces albus, Rhodobacter capsulatus, Micromonospora echinofusca*;
the 4-coumarate-3-hydroxylase is from *Saccharothrix espanaensis, Streptomyces lunaelactis, Nocardia farcinica, Rhodococcus ruber;*
the caffeic acid O-methyltransferase is from *Triticum aestivum, Hordeum vulgare, Festuca arundinacea, Lolium perenne*; and/or
the pyridine nucleotide transhydrogenase is from *Escherichia coli (Escherichia coli MG1655).*

4. The recombinant cell according to claim 1, wherein, the recombinant cell comprises a prokaryotic cell or a eukaryotic cell; preferably, the prokaryotic cell comprises *E. coli, Bacillus subtilis* or *Streptomyces,* or the eukaryotic cell comprises a fungal cell, a yeast cell, an insect cell or a mammalian cells; more preferably, the recombinant cell is *E. coli*; more preferably, the *E. coli* is JM109 (DE3).

5. The recombinant cell according to claim 1, wherein, in the expression cassette(s) of tyrosine ammonia-lyase, 4-coumarate-3-hydroxylase, caffeic acid O-methyltransferase, the promoter(s) comprises a promoter selected from the group consisting of: a T7 promoter, a T5 promoter, a trc promoter; preferably comprises a T7 promoter; and/or
the replicon(s) comprises a replicon selected from the group consisting of: pBR322, p15a, pSC101; preferably, comprises pBR322.

6. The recombinant cell according to claim 2, wherein, in the expression cassette of pyrimidine nucleotide hydrogenase, the promoter comprises a promoter selected from the group consisting of: a T7 promoter, a T5 promoter; preferably comprises a T7 promoter; or
the operon comprises a operon selected from the group consisting of: T7 operon, T5 operon; or
the replicon comprises SC101.

7. Use of the recombinant cell according to any one of claims 1 to 6 for the production of ferulic acid; preferably, for the conversion of L-tyrosine to ferulic acid.

8. A method for producing ferulic acid, wherein the method comprises:
(1) providing the recombinant cell according to any one of claims 1 to 6; and
(2) culturing the recombinant cell of (1) to produce ferulic acid.

9. The method according to claim 8, wherein the recombinant cell is a prokaryotic cell using glycerol as a carbon source to produce ferulic acid; preferably, the culture medium of the cell comprises glycerol of 0.5% to 8% by volume, preferably 1% to 6%, more preferably 1.5% to 4%.

10. The method according to claim 8, wherein, in step (2), said culturing the recombinant cell of (1) is in a culture system containing L-tyrosine.

11. An expression cassette or recombinant construct comprising nucleic acids encoding a group of enzymes comprising tyrosine ammonia-lyase, 4-coumarate-3-hydroxylase and caffeic acid O-methyltransferase; preferably, the group of enzymes also comprises pyridine nucleotide transhydrogenase.

12. The expression cassette or recombinant construct according to claim 11, wherein, in the expression cassette(s) of tyrosine ammonia-lyase, 4-coumarate-3-hydroxylase, caffeic acid O-methyltransferase, the promoter(s) comprises a promoter selected from the group consisting of: a T7 promoter, a T5 promoter, a trc promoter; preferably comprises a T7 promoter; or the replicon(s) comprises a replicon selected from the group consisting of:
pBR322, p15a, pSC101; preferably, comprises pBR322; or
in the expression cassette of pyrimidine nucleotide hydrogenase, the promoter(s) comprises a promoter selected from the group consisting of: a T7 promoter, a T5 promoter;
preferably comprises a T7 promoter; the replicon(s) comprises a replicon selected from the group consisting of: SC101, p15a, pBR322.

13. Use of the expression cassette or recombinant construct according to claim 11 or 12 in the manufacture of a recombinant cell for producing ferulic acid.

14. Use of a group of enzymes or the coding nucleic acids or expression cassette of the group of enzymes, for expressing and producing ferulic acid in a recombinant cell; or in the manufacture of a recombinant cell for producing ferulic acid; the group of enzymes comprises tyrosine ammonia-lyase, 4-coumarate-3-hydroxylase and caffeic acid O-methyltransferase; preferably, the group of enzymes also comprises pyridine nucleotide transhydrogenase.

15. A kit for the production of ferulic acid, wherein, the kit comprises:
the recombinant cell according to any one of claims 1 to 6; or
the expression cassette or recombinant construct according to claim 11 or 12;
preferably, the kit also comprises a host cell.

16. The kit according to claim 15, wherein, the kit also comprises L-tyrosine and/or basic culture medium.


SEQUENCE LISTING

<110>  CAS CENTER FOR EXCELLENCE IN MOLECULAR PLANT SCIENCES

<120>  Engineering bacteria for ferulic acid production, preparation method
and use thereof

<130>  202951

<160>  24

<170>  SIPOSequenceListing 1.0

<210>  1
<211>  28
<212>  DNA
<213>  primer

<400>  1
cgggaattcg cgcaaaaaac ccctcaag                                          28

<210>  2
<211>  30
<212>  DNA
<213>  primer

<400>  2
ctttactaag ctgacgatag tcatgccccg                                        30

<210>  3
<211>  32
<212>  DNA
<213>  primer

<400>  3
gactatcgtc agcttagtaa agccctcgct ag                                     32

<210>  4
<211>  20
<212>  DNA
<213>  primer

<400>  4
gttttttgcg cgaattcccg                                                   20

<210>  5
<211>  19
<212>  DNA
<213>  primer

<400>  5
agatctcgat cctctacgc                                                    19

<210>  6
<211>  28
<212>  DNA
<213>  primer

<400>  6
tctagaaata attttgttta actttaag                                          28

<210>  7
<211>  33

<212>  DNA
<213>  primer

<400>  7
caaaattatt tctagaatag ttaatttctc ctc                                    33

<210>  8
<211>  38
<212>  DNA
<213>  primer

<400>  8
gtagaggatc gagatctaaa tcataaaaaa tttatttg                               38

<210>  9
<211>  42
<212>  DNA
<213>  primer

<400>  9
caaaagcacc gccggacatc agcgctagcg gagtgtatac tg                          42

<210>  10
<211>  36
<212>  DNA
<213>  primer

<400>  10
catgactaac atgagaatta caacttatat cgtatg                                 36

<210>  11
<211>  36
<212>  DNA
<213>  primer

<400>  11
catacgatat aagttgtaat tctcatgtta gtcatg                                 36

<210>  12
<211>  42
<212>  DNA
<213>  primer

<400>  12
cagtatacac tccgctagcg ctgatgtccg gcggtgcttt tg                          42

<210>  13
<211>  30
<212>  DNA
<213>  primer

<400>  13
ccaagcttac tagtttacat gttttcgatg                                        30

<210>  14
<211>  25
<212>  DNA
<213>  primer

<400>  14
catgccatgg aaagtaaagt agttg                                             25

<210>  15

<211>  30
<212>  DNA
<213>  primer

<400>  15
ccaagcttac tagtttaatc cagccattcg                                        30

<210>  16
<211>  28
<212>  DNA
<213>  primer

<400>  16
ggaattccat atgtccaagc aacagatc                                          28

<210>  17
<211>  34
<212>  DNA
<213>  primer

<400>  17
cccaagctta ctagtttact caaactcatt ccag                                   34

<210>  18
<211>  25
<212>  DNA
<213>  primer

<400>  18
catgccatgg cggtaacgca aacag                                             25

<210>  19
<211>  35
<212>  DNA
<213>  primer

<400>  19
cccaagctta ctagtttaca gagctttcag gattg                                  35

<210>  20
<211>  24
<212>  DNA
<213>  primer

<400>  20
catgccatgg aagggaatat catg                                              24

<210>  21
<211>  47
<212>  DNA
<213>  primer

<400>  21
ctttaagaag gagatataca tgtttgaaaa tatatcatca aatggag                     47

<210>  22
<211>  48
<212>  DNA
<213>  primer

<400>  22
gtgcggccgc aagcttgtcg attataggtt taaaactatt gatttatg                    48

<210>  23
<211>  48
<212>  DNA
<213>  primer

<400>  23
cataaatcaa tagttttaaa cctataatcg acaagcttgc ggccgcac                    48

<210>  24
<211>  47
<212>  DNA
<213>  primer

<400>  24
ctccatttga tgatatattt tcaaacatgt atatctcctt cttaaag                     47